# EUROPÄISCHE PATENTANMELDUNG

(11) **EP 4 390 009 A1**
(43) Veröffentlichungstag der Anmeldung: **26.06.2024**
(21) Anmeldenummer: 23216242.0
(22) Anmeldetag: 13.12.2023
(51) Int. Cl.: E04G 21/08, G01N 33/38

(54) **SYSTEM ZUM FÜHREN EINER BEDIENPERSON BEI DER BETONVERDICHTUNG**

(30) Priorität: 21.12.2022 DE 102022134330
(71) Anmelder: Wacker Neuson Produktion GmbH & Co. KG, 85084 Reichertshofen (DE)
(72) Erfinder: LANGE, Christian, 85051 Ingolstadt (DE); BERGER, Rudolf, 82031 Grünwald (DE); DILLER, Patrick, 86558 Hohenwart (DE); WEISKOPF, Alto, 85250 Altomünster (DE)
(74) Vertreter: Müller Hoffmann & Partner

(57) **Zusammenfassung**

Es wird ein System zum Führen einer Bedienperson (1) bei einer Betonverdichtung mit einer Betonverdichtungsvorrichtung (2) angegeben, mit
- einer Planungsvorrichtung zum Speichern von Planungsdaten, wobei die Planungsdaten dienen zum Definieren von Orten (15) in einem definierten Gebiet (14), an denen eine Betonverdichtung mit der Betonverdichtungsvorrichtung zu erfolgen hat, und zum Definieren einer Reihenfolge der Orte (15), in der die Betonverdichtung zu erfolgen hat;
- einer Positionsbestimmungsvorrichtung zum Bestimmen der jeweils aktuellen Position der Betonverdichtungsvorrichtung (2); und mit
- einer Anzeigeeinrichtung (13) zum Anzeigen von wenigstens jeweils dem Ort (15) in der Reihenfolge von Orten, an dem aktuell eine Betonverdichtung zu erfolgen hat.

## Beschreibung

Die Erfindung betrifft ein System zum Führen einer Bedienperson bei der Betonverdichtung.

Frisch vergossener, noch fließfähiger Beton muss verdichtet werden, um Gaseinschlüsse aus dem Beton zu lösen und Kiesnester zu vergleichmäßigen. Dadurch wird die Qualität und Festigkeit des Betons erhöht. Vielfältige technische Regelwerke enthalten entsprechende Vorgaben für die Betonverdichtung.

Zur Betonverdichtung werden häufig mobile, d.h. tragbare, Innenrüttler (Innenvibratoren) eingesetzt. Derartige Innenrüttler weisen eine Rütteleinheit zum Erzeugen einer Vibration bzw. Schwingung auf, die in den noch fließfähigen Beton eingeleitet wird, um den Beton zu verdichten. Die Rütteleinheit kann z.B. einen Unwuchterreger und einen den Unwuchterreger antreibenden Elektromotor aufweisen, die in einem gemeinsamen Gehäuse, z.B. einer Rüttelflasche, angeordnet sind. Weiterhin kann eine Bedieneinheit, z.B. ein Schaltergehäuse, ein Frequenzumformer oder Ähnliches als Teil des Innenrüttlers vorgesehen sein. Die Rütteleinheit und die Bedieneinheit können durch einen robusten Schutz- und Bedienungsschlauch miteinander verbunden sein. Im Inneren des Schutz- und Bedienungsschlauchs werden die elektrischen Leitungen für den Antrieb in der Rütteleinheit geführt. Eine Bedienperson kann die Rütteleinheit durch Greifen des Schutz- und Bedienungsschlauchs führen und zielgerichtet an den gewünschten Stellen in den zu verdichtenden Beton eintauchen.

Es sind auch Innenrüttler bekannt, bei denen der Elektroantrieb nicht in der Rüttelflasche, sondern außerhalb angeordnet ist. In diesem Fall wird die Drehbewegung des Elektroantriebs über eine biegsame Welle auf den Unwuchterreger in der Rütteleinheit übertragen. Die biegsame Welle kann ebenfalls im Inneren des Schutz- und Bedienungsschlauchs angeordnet sein.

Der Elektroantrieb kann auf einer Baustelle über elektrischen Strom aus einem öffentlichen Netz oder aus einem speziellen Baustellennetz versorgt werden. Mithilfe eines Frequenzumformers kann der über das Netz bezogene Strom in einen hinsichtlich Spannung, Stromart (Wechselstrom) und Frequenz für den Elektroantrieb geeigneten Strom gewandelt werden.

In jüngerer Zeit sind auch Innenrüttler bekannt geworden, die ihre elektrische Energie über einen Akku (elektrischer Energiespeicher) beziehen. Der Akku kann z.B. in einem Rucksack-Tragesystem angeordnet sein, das von einer Bedienperson auf dem Rücken getragen werden kann. In dem Rucksack-Tragesystem kann neben dem Akku auch ein Frequenzumrichter zum Erzeugen des für den Elektroantrieb erforderlichen Stroms, z.B. Drehstroms, angeordnet sein. Das Rucksack-Tragesystem ermöglicht eine hohe Mobilität des Innenrüttlers, ohne dass eine elektrische Verbindung zu einem Netz erforderlich ist. Ein Beispiel für ein derartiges Rucksack-Tragesystem ist in der DE 10 2018 118 552 A1 beschrieben.

Die Betonverdichtung mit Innenrüttlern erfolgt in der Praxis meistens aufgrund von Annahmen oder aufgrund der Erfahrung des Anwenders. Um die richtige Verdichtungsdauer zu erreichen und dem Anwender zu signalisieren, dass eine ausreichende Verdichtung des Betons erreicht wurde, wurden Verfahren entwickelt, die z.B. in den nachveröffentlichten Patentanmeldungen DE 10 2022 118 541 A1 und DE 10 2022 118 542 A1 beschrieben sind. Auf diese Weise erfährt der Anwender, dass an dem Ort der aktuellen Verdichtung ausreichend verdichtet wurde. Die Wahl des Verdichtungsortes, insbesondere die Wahl des Eintauchortes, an dem die Rüttelflasche des Innenrüttlers in den zu verdichtenden Beton eingetaucht wird, erfolgt allerdings nach wie vor basierend auf Erfahrungswerten oder Annahmen des Anwenders.

Auch bei einer vorherigen Planung der Betonage wird der Anwender, der den Innenrüttler führt, keinen Plan in Papierform o.Ä. mit sich führen, da er beide Hände zur Bedienung des Innenrüttlers benötigt.

Speziell bei größeren Betonagen, beispielsweise Hallenböden oder Deckenbetonagen, muss eine ausreichende Verdichtung gewährleistet und gegebenenfalls auch dokumentiert werden. Zwar kann es möglich sein, den Ort der Verdichtung im Beton zu bestimmen und zu dokumentieren. Jedoch erhält die Bedienperson keine Rückmeldung, ob bestimmte Stellen verdichtet oder ausgelassen wurden. Auch erhält die Bedienperson keine Information darüber, wo als nächstes verdichtet werden soll, um den Verdichtungsvorgang möglichst effizient zu gestalten. Die Verdichtung hängt somit allein von der Erfahrung und Sorgfalt der Bedienperson ab.

Der Erfindung liegt die Aufgabe zugrunde, eine Bedienerführung für die Betonverdichtung unter Berücksichtigung des Ortes der Verdichtung sowie des Verdichtungsfortschritts bei einem jeweiligen Eintauchvorgang zu ermöglichen.

Die Aufgabe wird erfindungsgemäß durch ein System mit den Merkmalen von Anspruch 1 sowie durch ein Verfahren mit den Merkmalen des nebengeordneten Anspruchs gelöst. Vorteilhafte Ausgestaltungen sind in den abhängigen Ansprüchen angegeben.

Es wird ein System zum Führen einer Bedienperson bei einer Betonverdichtung mit einer Betonverdichtungsvorrichtung angegeben, mit einer Planungsvorrichtung zum Speichern von Planungsdaten, wobei die Planungsdaten dienen zum Definieren von Orten in einem definierten Gebiet, an denen eine Betonverdichtung mit der Betonverdichtungsvorrichtung zu erfolgen hat; und mit einer Positionsbestimmungsvorrichtung zum Bestimmen der jeweils aktuellen Position der Betonverdichtungsvorrichtung; und mit einer Anzeigeeinrichtung zum Anzeigen von jeweils demjenigen Ort, an dem aktuell eine Betonverdichtung zu erfolgen hat.

Das System kann als Assistenzsystem eine Bedienperson bei der Betonverdichtung im Rahmen eines Betonagevorgangs unterstützen, indem der Bedienperson Hinweise gegeben werden, an welchem Ort eine jeweilige Betonverdichtung jeweils aktuell erfolgen sollte. Die Verdichtungsorte können insbesondere Orte sein, an denen die Betonverdichtungsvorrichtung in den zu verdichtenden Beton eingetaucht werden soll.

Bei einer Variante kann der Bedienperson auch eine Reihenfolge der Orte der Betonverdichtung vorgegeben werden. Dabei können die Planungsdaten auch zum Definieren einer Reihenfolge der Orte dienen, in der die Betonverdichtung zu erfolgen hat, wobei die Anzeigeeinrichtung ausgebildet sein kann zum Anzeigen von jeweils demjenigen Ort in der Reihenfolge von Orten, an dem aktuell eine Betonverdichtung zu erfolgen hat.

Somit können Orte definiert werden, in denen verdichtet werden soll, wobei mindestens ein Ort angezeigt wird, an dem verdichtet werden soll.

Die Bedienperson kann sich dann während des Betonageprozesses an die vorgegebene Reihenfolge der Verdichtungsorte halten. Diese Vorgabe einer Reihenfolge kann den Vorteil haben, dass damit eine Wegoptimierung verbunden sein kann, damit die Betonage zeit- und kostenoptimiert verrichtet werden kann. Alternativ kann die Bedienperson aber auch davon abweichen und eine eigene Verdichtungsreihenfolge wählen.

Die Bedienperson kann z.B. bei einer Variante eine Art Karte mit Orten, an denen verdichtet werden soll, angezeigt bekommen und diese Orte jedoch nach eigener Auswahl abarbeiten und so zum gewünschten Verdichtungsziel kommen. Falls dabei Orte ausgelassen werden, könnte dies als Verdichtungsfehlstellen angezeigt werden, z.B. in einer "Heatmap". Dadurch kann die Bedienperson erkennen, welche Stellen noch verdichtet werden müssen.

Es ist also möglich, dass je nach Variante oder Betriebsart der Bedienperson jeweils nur ein Verdichtungsort, mehrere Verdichtungsorte (ohne festgelegte Reihenfolge) oder mehrere Verdichtungsorte (mit festgelegter bzw. vorgeschlagener Reihenfolge) angezeigt werden.

Als Betonverdichtungsvorrichtung eignet sich insbesondere ein an sich bekannter Innenrüttler, bei dem eine Rüttelflasche in den zu verdichtenden Beton eingetaucht wird. Die Rüttelflasche umschließt einen von einem Elektromotor angetriebenen Unwuchterreger und wird über einen daran angeschlossenen Schutz- und Bedienungsschlauch von der Bedienperson geführt. Bei einer Variante ist der Elektromotor außerhalb der Rüttelflasche angeordnet und wir z.B. von der Bedienperson getragen. Die Drehbewegung des Elektromotors kann dann über eine biegsame Welle auf den Unwuchterreger in der Rüttelflasche übertragen werden.

Das für die Planungsvorrichtung relevante definierte Gebiet kann z.B. eine (Ober-) Fläche mit zu verdichtendem Beton, z.B. innerhalb einer Schalung sein. Insbesondere kann das definierte Gebiet die Betonoberfläche sein, in die die Bodenverdichtungsvorrichtung eingetaucht werden soll. Dabei kann die Betonoberfläche als Ganzes erfasst oder auch in Teilbereiche aufgeteilt werden, die jeweils als definiertes Gebiet verstanden werden können.

Als Orte können die jeweiligen Eintauchstellen für die Rüttelflasche eines Innenrüttlers verstanden werden. Insbesondere können die Orte innerhalb einer Fläche (z.B. der Betonoberfläche) mit Koordinaten der optimalen Eintauchpunkte versehen werden.

Die Reihenfolge von Orten entspricht der Reihenfolge der Eintauchstellen, an denen nacheinander die Rüttelflasche in den zu verdichtenden Beton eingetaucht und der Beton somit verdichtet werden soll.

Die Anzeigeeinrichtung zeigt dem Bediener an, an welcher Stelle er aktuell eine Betonverdichtung vornehmen soll. Dies ist z.B. möglich durch entsprechende Handlungsanweisungen, z.B. dass der Bediener den Innenrüttler um einen bestimmten Weg nach vorne oder zur Seite bewegen soll. Der Bediener kann diesen Handlungsempfehlungen folgen und somit leicht den Innenrüttler an der durch die Planungsvorrichtung vorgegebenen Stelle in den Beton eintauchen. Der Bediener kann somit auf der Anzeigeeinrichtung sehen, wo er derzeit verdichten soll.

Die Anzeigeeinrichtung kann ein Display aufweisen, auf dem der Bediener den Ort direkt sehen kann oder über das er eine entsprechende Handlungsanweisung erhält, um den Ort zu erreichen. Zum Beispiel kann die Anzeigeeinrichtung ein Smartphone sein, ein Tablet, eine Smartwatch o.ä. Ebenfalls ist es möglich, eine VR-Brille (Virtual Reality) oder eine AR-Brille (Augmented Reality) zu nutzen. Ebenfalls ist die Nutzung eines Head-up Displays möglich.

Die Anzeigeeinrichtung kann ausgebildet sein zum Anzeigen der Position der Betonverdichtungsvorrichtung und/oder zum Anzeigen von wenigstens jeweils demjenigen Ort in der Reihenfolge von Orten, an dem als nächstes eine Betonverdichtung zu erfolgen hat. Die Bedienperson kann somit auf der Anzeigeeinrichtung erkennen, inwieweit die Positionen von Innenrüttler und Eintauchort voneinander abweichen. Damit kann die Bedienperson durch Bewegen des Innenrüttlers leicht die Abweichung verringern und an der vorgesehenen Stelle verdichten. Dabei ist es möglich, nicht nur die aktuell vorgesehene Eintauchstelle, sondern auch die nächste oder auch die weiteren Eintauchstellen anzuzeigen. Damit erkennt die Bedienperson auch die Sinnfälligkeit der von der Planungsvorrichtung vorgegebenen Planungsdaten.

Die Anzeigeeinrichtung kann ausgebildet sein zum Anzeigen des Ortes, an dem aktuell oder als nächstes eine Betonverdichtung zu erfolgen hat, in Relation zu der aktuellen Position der Betonverdichtungsvorrichtung. Dabei kann die aktuelle Position des Innenrüttlers angezeigt werden, in Relation zum aktuellen und/oder zum nächsten Verdichtungsort.

Es kann eine Verdichtungskoordinaten-Vorgabevorrichtung vorgesehen sein zum Erzeugen von Planungsdaten, die in der Planungsvorrichtung speicherbar sind. Die Verdichtungskoordinaten-Vorgabevorrichtung kann separat vorgesehen sein, z.B. in Form eines Laptops oder sogar ortsfern in einem größeren Datennetzwerk. Sie muss nur bedarfsweise mit der Planungsvorrichtung verbunden sein, um die (gegebenenfalls extern) erzeugten Planungsdaten zu übermitteln. Die Planungsdaten können neben den Eintauch- bzw. Verdichtungsorten auch Informationen zur jeweiligen Verdichtungsdauer bzw. zum anzustrebenden Verdichtungsgrad umfassen. Die Parameter Verdichtungsdauer bzw. Verdichtungsgrad geben einen Hinweis darauf, dass der Beton an der entsprechenden Stelle ausreichend verdichtet wurde.

Es kann eine Bestätigungseinrichtung vorgesehen sein, zum Bestätigen, dass an dem jeweiligen Ort, an dem aktuell eine Betonverdichtung erfolgt, eine ausreichende Betonverdichtung erreicht wurde, wobei die Bestätigungseinrichtung ausgebildet sein kann zum Markieren des nächsten Ortes in der Reihenfolge von Orten, an dem als nächstes eine Betonverdichtung zu erfolgen hat. Auf diese Weise können die Orte nacheinander abgearbeitet, also verdichtet werden, und zwar insbesondere in der von der Planungsvorrichtung vorgegebenen Reihenfolge. Die Verdichtungsreihenfolge hängt somit nicht von der Erfahrung oder Intuition der Bedienperson ab, sondern kann effizient geplant und von außen vorgegeben werden.

Die Bestätigungseinrichtung kann z.B. dazu dienen, dass die Bedienperson die Orte nacheinander wegklickt, wenn dort ausreichend verdichtet wurde. Dies kann auch über die Anzeigeeinrichtung angezeigt werden. Dabei kann die Anzeigeeinrichtung auch auf den nächsten Ort in der vorgegebenen Reihenfolge umschalten, wenn der aktuelle Ort als ausreichend verdichtet "weggeklickt" wurde.

Die Bestätigungseinrichtung kann manuell von einer Bedienperson betätigbar sein, um zu bestätigen, dass ausreichend verdichtet wurde. Ergänzend oder alternativ kann die Bestätigungseinrichtung auch automatisch betätigbar sein, wenn eine Verdichtungserkennungsvorrichtung erkannt hat, dass an dem aktuellen Ort durch die Betonverdichtungsvorrichtung ausreichend verdichtet wurde.

Hierzu ist z.B. in den nachveröffentlichten Patentanmeldungen DE 10 2022 118 541 A1 und DE 10 2022 118 542 A1 beschrieben, wie eine ausreichende Verdichtung in dem zu verdichtenden Beton erkannt werden kann.

Demnach können z.B. aufgrund eines gemessenen Strombezugs des den Unwuchterreger antreibenden Elektromotors Rückschlüsse auf die Verdichtung und damit auf den Verdichtungsfortschritt in dem zu verdichtenden Medium gezogen werden. Insbesondere können die erfassten Strommesswerte und Strommesswertverläufe in Relation zu vorbekannten Kriterien und Profilen gesetzt und daraus Rückschlüsse gezogen werden. Dies ist auch durch Auswertung der Stromgradienten Änderung der Stromaufnahme über die Zeit) möglich.

Mithilfe jeweils abgetasteter Stromwerte und jeweiliger Gradienten der Stromwerte bzw. der Tendenz der Stromwertentwicklung lässt sich erkennen, in welchem Arbeitszustand sich der Innenrüttler jeweils befindet. Während eines Verdichtungsvorgangs kann aufgrund des sich so ergebenden Stromprofils, also der Stromwerte und des Gradientenverlaufs, der Verdichtungszustand des Betons erkannt und z.B. mit Grenzwerten vergleichen. Wenn ein bestimmter Grenzwert erreicht ist, wird dies als Kriterium gewertet, dass der Beton an dieser Stelle ausreichend verdichtet wurde.

Für das präzise Führen der Bedienperson ist es erforderlich, die Position der Betonverdichtungsvorrichtung, also insbesondere die Position der Rütteleinheit, z.B. der Rüttelflasche, präzise zu erfassen. Zu diesem Zweck kann die Positionsbestimmungsvorrichtung in geeigneter Weise ausgebildet sein.

Insbesondere kann die Positionsbestimmungsvorrichtung ausgebildet sein als Vorrichtung zum Bestimmen der Position einer durch eine Bedienperson führbaren Rütteleinheit zur Betonverdichtung angegeben, mit einer Flächen-Positionsbestimmungseinrichtung mit einer Empfangseinrichtung, wobei die Flächen-Positionsbestimmungseinrichtung ausgebildet ist zum Bestimmen der Position der Empfangseinrichtung in der Ebene; mit einer Ausrichtungsbestimmungseinrichtung zum Bestimmen einer Ausrichtung einer Arbeitsrichtung der Bedienperson; und mit einer Korrektureinrichtung zum Korrigieren der Position der Empfangseinrichtung mit einem Offset in Richtung der Ausrichtung der Arbeitsrichtung und damit zum Bestimmen der Position der Rütteleinheit in der Ebene.

Die Flächen-Positionsbestimmungseinrichtung dient zum Bestimmen der Position bzw. zum Lokalisieren der Empfangseinrichtung. Die Flächen-Positionsbestimmungseinrichtung ist somit in der Lage, den Ort der Empfangseinrichtung in der Fläche bzw. in der Ebene wenigstens also zweidimensional zu bestimmen.

Die Korrektureinrichtung dient zum Korrigieren der durch die Flächen-Positionsbestimmungseinrichtung bestimmten Position der Empfangseinrichtung mit Hilfe eines Offsets, wobei dabei die durch die Ausrichtungsbestimmungseinrichtung bestimmte Ausrichtung der Arbeitsrichtung berücksichtigt wird. Der Offset stellt einen Korrekturwert in Richtung der von der Ausrichtungsbestimmungseinrichtung bestimmten Ausrichtung und damit der Ausrichtung der Bedienperson dar. Auf diese Weise wird mit Hilfe des Offsets berücksichtigt, dass die Rütteleinheit in der Ebene nicht direkt an der Empfangseinrichtung angeordnet ist, sondern ein Stück entfernt.

Die Flächen-Positionsbestimmungseinrichtung kann wenigstens ein Ortungssystem aufweisen, ausgewählt aus der Gruppe RTK (Real Time Kinematic - Echtzeitkinematik), DGPS (Differential Global Positioning System - Differentielles globales Positionierungssystem), UWB (Ultra-wide Band - Ultrabreitband). Derartige Ortungssysteme ermöglichen eine sehr hohe Genauigkeit im Bereich von wenigen Zentimetern in der Horizontalen. Als Ortungssysteme sind weiterhin Bluetooth-Funkeinrichtungen oder optische Systeme, z.B. mit Bilderkennung möglich.

Es kann eine Tiefen-Positionsbestimmungseinrichtung vorgesehen sein, zum Bestimmen der Position der Rütteleinheit in der Tiefe. Die Tiefe kann dabei insbesondere in Bezug auf die Position der Empfangseinrichtung gemessen werden, also z.B. durch Messen des Abstands zur Empfangseinrichtung. Insbesondere kann dabei die z-Koordinate des Abstands ermittelt werden, um ein Maß für die Tiefe relativ zu der Empfangseinrichtung zu erhalten. Auch andere Messverfahren sind möglich, um die Tiefenposition der Rütteleinheit zu bestimmen.

Auf diese Weise kann zusätzlich zu der oben beschriebenen zweidimensionalen Positionserfassung in der Fläche bzw. Ebene (Flächenposition) auch eine dritte Dimension (Tiefenposition) bestimmt werden. Damit kann die Position der Rütteleinheit dreidimensional bestimmt werden, um auch bei tiefen Betonbauteilen, z.B. Wänden, den Ort der Rütteleinheit zu erfassen.

Es kann eine Vergleichseinrichtung vorgesehen sein zum Vergleichen der aktuellen Position der Betonverdichtungsvorrichtung mit wenigstens dem aktuellen ersten Ort der vorgegebenen Reihenfolge von Orten, an dem als nächstes eine Betonverdichtung erfolgen soll und zum Erkennen einer Abweichung der Position der Betonverdichtungsvorrichtung von dem vorgegebenen Ort; und mit einer Führungseinrichtung zum Definieren einer Bewegungsmaßnahme zum Bewegen der Betonverdichtungsvorrichtung, wobei mit der Bewegungsmaßnahme die Abweichung verringerbar ist; wobei die Anzeigeeinrichtung ausgebildet ist zum Anzeigen der Bewegungsmaßnahme für die Bedienperson.

Die Bedienperson erhält somit über die Anzeigeeinrichtung eine Empfehlung für eine geeignete Bewegungsmaßnahme, um die Abweichung zwischen der Eintauchposition bzw. Verdichtungsposition einerseits und der Position der Betonverdichtungsvorrichtung (z.B. der Rüttelflasche) zu verringern. Zum Beispiel kann die Bedienperson den Innenrüttler verlagern oder die Eintauchstelle ändern. Dabei können über die Anzeigeeinrichtung Hinweise, wie z.B. Pfeile, oder Streckenangaben angezeigt werden, wie die Bedienperson den Innenrüttler bewegen soll.

Es kann eine Annäherungserkennungsvorrichtung vorgesehen sein, zum Erkennen eines Zustands, in dem die Abweichung geringer ist als ein vorgegebener Abweichungsgrenzwert und zum Erzeugen eines Bestätigungssignals. Der Abweichungsgrenzwert definiert eine zulässige Abweichung zwischen dem (theoretisch) vorgegebenen "idealen" Eintauchort und dem tatsächlichen Eintauchort, an dem die Rüttelflasche in den zu verdichtenden Beton eingetaucht wird. Wenn der Abweichungsgrenzwert unterschritten wird, kann ein Bestätigungssignal für die Bedienperson erzeugt werden, dass die Rüttelflasche an der korrekten Position eingetaucht wurde.

Die Planungsdaten können außer den Daten zum Definieren der Orte, an denen eine Betonverdichtung zu erfolgen hat, weitere Daten aufweisen, ausgewählt aus der Gruppe: Zeitdauer der Verdichtung an dem betreffenden Ort, Intensität der Verdichtung an dem betreffenden Ort, Schwingungsanzahl der Verdichtung an dem betreffenden Ort, Betontyp oder Betonsorte, Beton-Mischungsverhältnisse, Daten zu Armierungseisen (insbesondere Anordnung, Engstellen, geplante sog. Rüttelgassen), Schütthöhe des Betons, Außen- bzw. Umgebungstemperatur, Temperatur des Betons, Luftfeuchte. Dadurch kann detailliert festgelegt werden, wie stark an welchem Ort bzw. an welcher Eintauchstelle verdichtet werden soll. Dies kann insbesondere dann interessant sein, wenn das zu verdichtende Betonteil stark unterschiedlich konturiert ist, so dass an verschiedenen Orten unterschiedlich stark bzw. intensiv verdichtet werden muss.

Es wird ein Verfahren angegeben, zum Führen einer Bedienperson bei einer Betonverdichtung mit einer Betonverdichtungsvorrichtung, mit den Schritten:
- Speichern von Planungsdaten, wobei die Planungsdaten dienen zum Definieren von Orten in einem definierten Gebiet, an denen eine Betonverdichtung mit der Betonverdichtungsvorrichtung zu erfolgen hat, und zum Definieren einer Reihenfolge der Orte, in der die Betonverdichtung zu erfolgen hat;
- Bestimmen der jeweils aktuellen Position der Betonverdichtungsvorrichtung; und
- Anzeigen von wenigstens jeweils dem Ort in der Reihenfolge von Orten, an dem aktuell eine Betonverdichtung zu erfolgen hat und/oder dem Ort in der Reihenfolge von Orten, an dem als nächstes eine Betonverdichtung zu erfolgen hat.

Diese und weitere Merkmale und Vorteile der Erfindung werden nachfolgend anhand von Beispielen unter Zuhilfenahme der begleitenden Figuren näher erläutert. Es zeigen:
- **Fig. 1**: eine schematische Draufsicht auf eine Positionsbestimmungsvorrichtung zum Bestimmen der Position einer durch eine Bedienperson führbaren Rütteleinheit zur Betonverdichtung;
- **Fig. 2**: die Positionsbestimmungsvorrichtung von Fig. 1 in der Seitenansicht;
- **Fig. 3**: die Positionsbestimmungsvorrichtung von Fig. 1 in der Seitenansicht, jedoch mit einem Innenrüttler mit längerem Schutz- und Bedienungsschlauch;
- **Fig. 4**: die Integration der Positionsbestimmungsvorrichtung in einem Assistenzsystem;
- **Fig. 5**: eine Draufsicht auf eine zu betonierende Fläche und ein Verdichtungsvorgang unter Nutzung des Assistenzsystems; und
- **Fig. 6**: eine Anzeigeeinrichtung des Assistenzsystems.

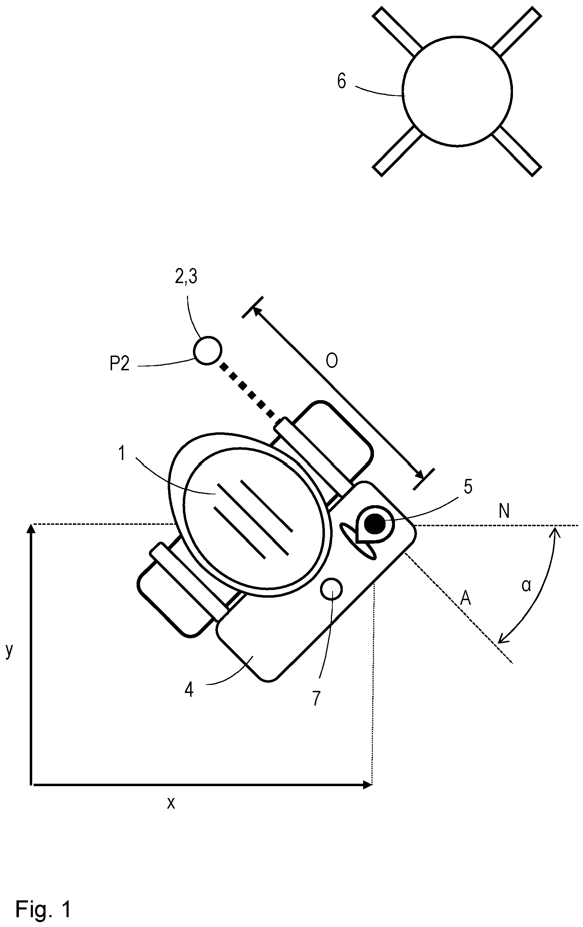

Fig. 1 zeigt eine schematische Draufsicht auf eine Bedienperson 1 (der Schutzhelm der Bedienperson 1 ist in der Draufsicht gut erkennbar), die einen Innenrüttler 2 zur Betonverdichtung führt. Von dem Innenrüttler 2 ist in Fig. 1 lediglich eine Rütteleinheit 3 dargestellt, die häufig auch als Rüttelflasche bezeichnet wird und einen nicht dargestellten Unwuchterreger zum Erzeugen der Verdichtungsvibrationen sowie einen Elektromotor zum Antreiben des Unwuchterregers enthält. Der Aufbau eines derartigen Innenrüttlers 2 ist bekannt und muss daher an dieser Stelle nicht weiter vertieft werden.

Die Rütteleinheit 3 wird vom Bediener in den zu verdichtenden Beton eingetaucht und für eine gewisse Zeit an einem Ort gehalten. Nachfolgend bewegt der Bediener die Rütteleinheit 3 an einen anderen Ort, um die Verdichtung an anderer Stelle fortzusetzen.

Zur Bestimmung des Verdichtungsfortschritts wurden verschiedene Verfahren entwickelt, die der Bedienperson anzeigen bzw. mitteilen, wie weit die Verdichtung an der betreffenden Stelle fortgeschritten ist bzw. ob eine ausreichende Verdichtung erreicht wurde (z.B. in Form eines Verdichtungsgrades). Beispielhaft sei auf die nachveröffentlichten Patentanmeldungen DE 10 2022 118 541 A1 und DE 10 2022 118 542 A1 verwiesen.

Der Innenrüttler 2 ist mit einer Positionsbestimmungsvorrichtung ausgestattet, mit deren Hilfe die Position der Rütteleinheit 3 mit hoher Genauigkeit im dreidimensionalen Raum bestimmt werden kann, wie später erläutert wird.

Der in der Rütteleinheit 3 befindliche Elektromotor wird durch einen nicht dargestellten elektrischen Energiespeicher bzw. Akku gespeist, der vom Bediener in einem Rucksack 4 auf dem Rücken getragen wird. In dem Rucksack 4 können neben dem Akku auch weitere Komponenten angeordnet sein, z.B. ein nicht dargestellter Frequenzumformer, mit dem der aus dem Akku bezogene elektrische Strom in einer für den Elektromotor in der Rütteleinheit 3 geeigneten Weise angepasst werden kann. Insbesondere wird dabei der aus dem Akku bezogene Gleichstrom in einen Wechselstrom bzw. Drehstrom gewandelt und hinsichtlich der Frequenz angepasst.

Weiterhin ist in dem Rucksack 4 ein als Empfangseinrichtung dienender Empfänger 5 angeordnet. Der Empfänger 5 ist derart ausgebildet, dass sein Ort mit hoher bzw. höchstmöglicher Genauigkeit bestimmt werden kann. Dazu können geeignete Ortungsverfahren genutzt werden, z.B. das Verfahren RTK (Real Time Kinematic - Echtzeitkinematik), DGPS (Differential Global Positioning System - Differenzielles globales Positionierungssystem) und/oder UWB (Ultra-Wideband - Ultrabreitband).

In dem in Fig. 1 gezeigten Beispiel erfolgt die Positionsmessung nach dem RTK-Verfahren mithilfe des als RTK-Empfänger ausgebildeten Empfängers 5 in Verbindung mit einer festen Basisstation 6, die auf der Baustelle in der Nähe des Arbeitsorts angeordnet ist.

Bei dem RTK-System können Genauigkeiten von 1 bis 2 cm in der Horizontalen erreicht werden. Die Koordinaten der Punkte werden nach der Initialisierung in Echtzeit berechnet. Die präzisen Positionen werden wie beim DGPS-System relativ zu Referenzstationen mit feststehenden Koordinaten, hier z.B. der Basisstation 6, bestimmt.

Das Ortungssystem zusammen mit dem Empfänger 5 und - soweit vorhanden - der Basisstation 6 bilden eine Flächen-Positionsbestimmungseinrichtung.

Die genaue Position des Empfängers 5 kann z.B. durch Koordinaten x, y in Bezug auf die Basisstation 6 bestimmt werden.

Mithilfe des Rucksacks 4 wird durch die Bedienperson 1 weiterhin eine Ausrichtungsbestimmungseinrichtung 7 getragen. Die Ausrichtungsbestimmungseinrichtung 7 kann auch an einem anderen Ort an der Bedienperson 1 angebracht werden. Sie dient dazu, die Ausrichtung der Bedienperson 1 relativ zu einem feststehenden Koordinatensystem, z.B. relativ zu dem magnetischen Norden, zu bestimmen. Aus der Ausrichtung der Bedienperson 1 kann unmittelbar auf deren Arbeitsrichtung geschlossen werden.

In dem Fig. 1 gezeigten Beispiel ist die gemessene Ausrichtung der Bedienperson 1 mit A gekennzeichnet, die sich gegenüber einer absoluten Ausrichtung, z.B. der Nordrichtung N, um den Winkel α unterscheidet. Mit der Ausrichtung A ist auch die Arbeitsrichtung und die Blickrichtung der Bedienperson 1 verbunden.

Die Ausrichtungsbestimmungseinrichtung 7 kann ein elektronisches Kompassmodul oder ein Magnetometer mit mehreren Freiheitsgraden aufweisen, um die räumliche Ausrichtung A der Bedienperson 1 zu erfassen.

Durch die mit Hilfe des Empfängers 5 und des Ortungssystems erfassten Position der Bedienperson 1 und der durch die Ausrichtungsbestimmungseinrichtung 7 erfassten Ausrichtung A der Bedienperson 1 kann die Position des Innenrüttlers, insbesondere der Rütteleinheit 3, mit hoher Genauigkeit errechnet werden. Dazu wird ein Offset O mit dem entsprechenden Winkel (z.B. α) auf die Position x, y, die für den Ort des Empfängers 5 am Rucksack 4 gemessen wurde, aufgerechnet. Der Offset O kann dabei z.B. die Armlänge der Bedienperson berücksichtigen, wobei auch eine schräge Armhaltung mit eingerechnet werden kann. Zudem kann der Abstand des Empfängers 5 vom Arm der Bedienperson 1 mit eingerechnet werden, der sich im in Fig. 1 gezeigten Beispiel im Rucksack 4 befindet und damit hinter dem Arm der Bedienperson 1.

Auf diese Weise lässt sich die zweidimensionale Position P2 in der Ebene bzw. Fläche und damit die zweidimensionale Position der Rütteleinheit 3 in der in Fig. 1 gezeigten Draufsicht mit hoher Genauigkeit bestimmen.

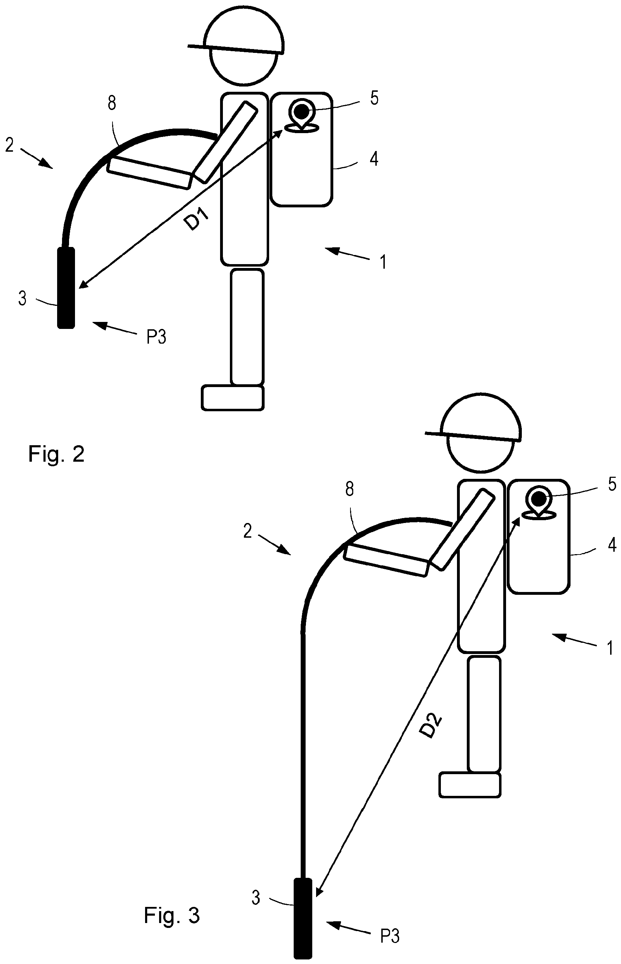

Zusätzlich wird bei der Positionsbestimmungsvorrichtung auch die Tiefe erfasst, sodass die dreidimensionale Position der Rütteleinheit 3 bestimmt werden kann. Dies wird anhand der Figuren 2 und 3 erläutert.

Die Figuren 2 und 3 zeigen die Bedienperson 1 in schematischer Seitenansicht, jeweils mit dem Innenrüttler 2. Der Innenrüttler 2 weist die Rütteleinheit 3 auf, die über einen Schutz- und Bedienungsschlauch 8 gehalten wird. Der Schutz- und Bedienungsschlauch 8 verbindet die Rütteleinheit 3 mit dem Akku im Rucksack 4. Im Inneren des Schutz- und Bedienungsschlauchs 8 verlaufen elektrische Leitungen, über die der Elektromotor in der Rütteleinheit 3 mit elektrischem Strom versorgt werden kann. Zudem ist der Schutz- und Bedienungsschlauch 8 dazu ausgebildet, von der Bedienperson 1 mit den Händen gehalten zu werden, wie in den Figuren 2 und 3 erkennbar. Die Schutz- und Bedienungsschläuche 8 weisen in den Figuren 2 und 3 eine unterschiedliche Länge auf.

Um die Position der Rütteleinheit 3 in der Tiefe zu bestimmen, weist die Positionsbestimmungsvorrichtung ein weiteres Messsystem auf.

Zur Bestimmung der Position der Tiefe der Rütteleinheit 3 ist eine Tiefen-Positionsbestimmungseinrichtung vorgesehen. Dabei wird insbesondere eine Tiefenkoordinate in Bezug auf den Empfänger 5 gemessen. Dies kann z.B. damit erreicht werden, dass der der Abstand der Rütteleinheit 3 zum Empfänger 5 bestimmt wird. In Fig. 2 ist dieser Abstand mit D 1, in Fig. 3 mit D2 gekennzeichnet. Aus dem Abstand D1, D2 und den weiteren, bereits oben erläuterten Koordinaten bzw. Abmessungen lässt sich eine Tiefenkoordinate in z-Richtung ableiten, die den relativen Höhenunterschied (Tiefe) der Rütteleinheit 3 zum Empfänger 5 bemisst.

Die Erfassung der Tiefe und damit der dritten Dimension der Position der Rütteleinheit 3 erlaubt es, die Position auch bei tiefen Betonteilen, z.B. Wänden, dreidimensional und damit mit hoher Genauigkeit zu erfassen.

Dazu wird der Abstand der Rütteleinheit 2 zum Empfänger 5 oder auch zu einer weiteren Sende- / Empfangsvorrichtung gemessen. Die weitere Sende-/Empfangseinrichtung kann in geeigneter Weise vom Bediener getragen werden und z.B. ebenfalls in dem Rucksack 4 untergebracht werden.

Die Bestimmung des Abstandes zwischen Innenrüttler und Empfänger 5 und bzw. der weiteren, nicht dargestellten Sende-/ Empfangsvorrichtung kann durch Auswertung der Signalstärke eines zwischen den Komponenten ausgetauschten Funksignals gemessen werden.

Bei tieferem Eintauchen der Rütteleinheit 3 in den zu verdichtenden Beton erhöht sich die Distanz D1, D2, sodass die Signalstärke sinkt. Dadurch kann ein Rückschluss auf die Eintauchtiefe erfolgen.

Die somit ermittelte Position der Rütteleinheit 3 kann beispielsweise an ein Mobilgerät oder entsprechendes Gateway gesendet werden und darüber für die Dokumentation des Betonagevorgangs erfasst werden.

Mit dem System wird die Möglichkeit geschaffen, eine Betonage vollständig zu dokumentieren und dabei die ermittelten Verdichtungsgrade nicht nur allgemein örtlich in der Fläche, sondern auch räumlich zuzuordnen. Zur Dokumentation können so genannte Heatmaps erstellt werden, die bei tieferen Bauteilen auch dreidimensional sein können.

Zudem stellt die präzise Erfassung des jeweiligen Ortes der Rütteleinheit 3 während des Betonagevorgangs, in Verbindung mit dem jeweiligen Verdichtungsgrad, eine Grundlage für Assistenzsysteme dar, die die Bedienperson 1 führen können. Insbesondere kann der Bedienperson 1 angezeigt werden, an welchen Stellen die Rütteleinheit 3 des Innenrüttlers 2 noch eingetaucht werden muss, um eine vollständige Verdichtung einer Betonage zu gewährleisten.

Ein Beispiel für ein derartiges Assistenzsystem wird nachfolgend erläutert.

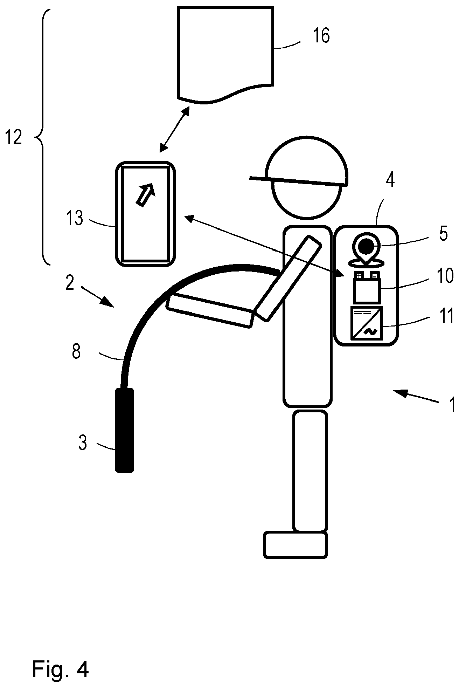

Fig. 4 zeigt die Bedienperson 1 mit dem Innenrüttler 2. Zusätzlich ist exemplarisch dargestellt, dass in dem Rucksack 4 neben dem Empfänger 5 ein als elektrischer Energiespeicher dienender Akku 10 und ein Frequenzumformer 11 zur Energieversorgung des Elektromotors im Innenrüttler 2 angeordnet sind.

Weiterhin ist ein Assistenzsystem 12 vorgesehen, zum Führen der Bedienperson 1 bei der Betonverdichtung. Das Assistenzsystem 12 kann eine oder mehrere Komponenten aufweisen, die insbesondere zur Planung der Position und Reihenfolge der Verdichtungsorte, zur Positionsbestimmung der aktuellen Position der Rütteleinheit 3 und zum Anzeigen des jeweils aktuellen Verdichtungsorts, an dem die Bedienperson 1 die Rütteleinheit 3 positionieren soll, dienen.

Als Teil des Assistenzsystems ist eine Anzeige 13 vorgesehen, auf der der Bedienperson 1 angezeigt wird, wohin sie die Rütteleinheit 3 des Innenrüttlers 2 bewegen soll, um die Rütteleinheit 3 an einer von dem Assistenzsystem 12 vorgegebenen Stelle (Eintauchort) positionieren und eintauchen zu können.

Zu diesem Zweck ist es mit Hilfe des Assistenzsystems 12 möglich, vorab Planungsdaten zu erstellen, an welchen Orten einer Betonoberfläche der noch fließfähige Beton verdichtet werden soll. Gleichzeitig kann die Reihenfolge der Verdichtungsorte festgelegt werden.

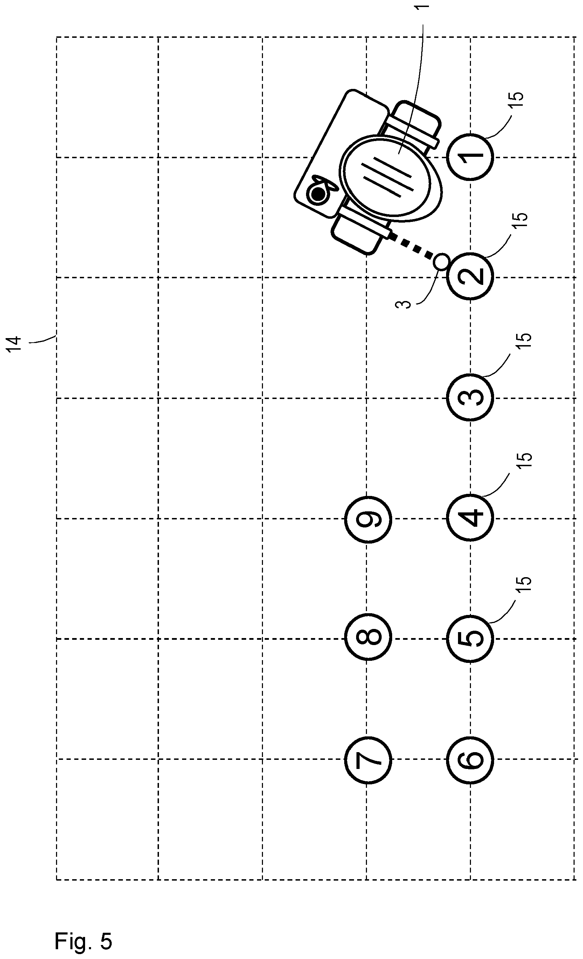

Fig. 5 zeigt in der Draufsicht ein Beispiel für die Betonage einer Fläche, z.B. eines Hallenbodens.

In dem Assistenzsystem 12 ist ein definiertes Gebiet 14 hinterlegt, das der Gesamtfläche des zu betonierenden Hallenbodens oder aber auch nur einer Teilfläche des Hallenbodens entsprechen kann.

Das definierte Gebiet 14 ist untergliedert in Verdichtungsorten 15, an denen mit Hilfe des Innenrüttlers 2 verdichtet werden soll. In dem gezeigten Beispiel sind die Verdichtungsorte 15 durchnummeriert mit 1, 2, 3, .. , 9. Auch die weiteren Bereiche des definierten Gebiets 14 können auf diese Weise als Verdichtungsorte 15 definiert werden.

In dem in Fig. 5 gezeigten Beispiel ist das definierte Gebiet 14 durch eine Gitterstruktur aufgeteilt und in die Verdichtungsorte 15 definiert. Selbstverständlich können die Verdichtungsorte 15 auch anders platziert oder sortiert werden.

Die Vorgabe des definierten Gebiets 14 und der Verdichtungsorte 15 kann extern, d.h. außerhalb vom Innenrüttler 2 erfolgen. Insbesondere kann diese Arbeit auch über ein Netzwerk oder mit Hilfe eines Laptops eingespielt werden. Die Vorgabe der Verdichtungsorte 15 kann von einem Experten vorgenommen werden, der vertieftere Kenntnis über die Betonverdichtung besitzt als die Bedienperson 1. Die Bedienperson 1 muss damit lediglich die Verdichtungsorte 15 nacheinander abfragen.

Zur Dokumentation der von einem Experten vorgenommenen Vorgabe oder auch zur automatischen Erarbeitung einer geeigneten Vorgabe der Verdichtungsorte 15 sowie deren Reihenfolge kann auch eine Verdichtungskoordinaten-Vorgabevorrichtung vorgesehen sein, die ein Planungssystem bildet, mit dem die Planungsdaten vorab erarbeitet werden können.

Zur Orientierung kann der Bedienperson 1 über die Anzeige 13 eine entsprechende Information vermittelt werden, wo aktuell der nächste Verdichtungsort 15 zu finden ist und wo die Bedienperson 1 dementsprechend die Rütteleinheit 3 eintauchen soll.

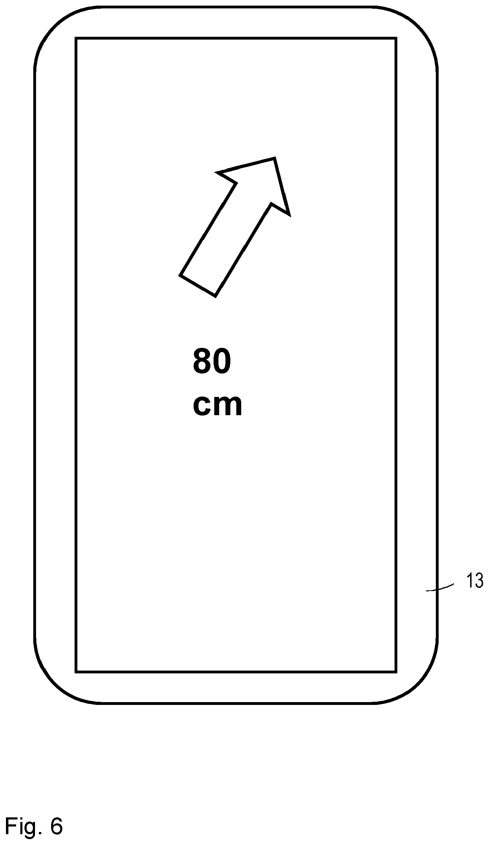

Fig. 6 zeigt beispielhaft ein als Anzeige 13 dienendes Smartphone, auf dem die Bedienperson 1 mit Hilfe eines Pfeils und einer Entfernungsangabe (hier: 80 cm) angezeigt wird, in welche Richtung und inwieweit sie die Rütteleinheit 3 noch bewegen muss, bevor die Rütteleinheit 3 die vorgegebene Position erreicht hat und in den zu verdichtenden Beton eintauchen kann.

Die Anzeige 13 ist nur exemplarisch dargestellt. Insbesondere muss die Anzeige 13 nicht in einem Smartphone oder Tablet untergebracht sein. Bei einer Variante ist es z.B. möglich, die Anzeige 13 in eine AR-Brille (Augmented Reality) zu integrieren und dem Bediener auf diese Weise direkt die Verdichtungsorte 15 anzuzeigen, die er nacheinander abarbeiten soll. Bei der Visualisierung der optimalen Verdichtungspunkte mit Hilfe einer AR-Brille können die Verdichtungspunkte den Bediener daher direkt auf der zu verdichtenden Betonoberfläche angezeigt werden.

Die Verdichtungsorte 15 können insoweit als optimale Verdichtungspunkte verstanden werden, die von dem Planungssystem vorab vorgegeben werden.

Beim Arbeitsbetrieb greift das Assistenzsystem 12 auf die jeweiligen Planungsdaten zurück und vergleicht diese mit der aktuellen Position der Bedienperson 1 bzw. des Innenrüttlers 2. Mit besonderer Genauigkeit kann dabei die Rütteleinheit 3 berücksichtigt werden.

Wenn sich die Bedienperson 1 an der optimalen Position oder in hinreichender Nähe befindet, erhält sie eine entsprechende Rückmeldung, z.B. über die Anzeige 13 und kann den Innenrüttler 2 eintauchen.

Wenn der Verdichtungsvorgang abgeschlossen ist, kann bei entsprechender Ausgestaltung des Assistenzsystems 12 die Bedienperson 1 eine Rückmeldung erhalten, dass ausreichend verdichtet wurde. Diese Rückmeldung kann z.B. taktil erfolgen, z.B. durch einen Ruck im Schutz- und Bedienungsschlauch 8. Alternativ oder ergänzend können auch akustische und/oder optische Signale erzeugt werden, die der Bedienperson 1 anzeigen, dass ausreichend verdichtet wurde.

Anschließend wird der Bedienperson 1 über die Anzeige 13 die Richtung und die Entfernung zum nächsten Verdichtungsort 15 angezeigt.

Während der Betonage kann somit auf der Anzeige 13 dokumentiert werden, ob an allen geplanten Punkten (Verdichtungsorte 15) oder in hinreichender Nähe dazu ausreichend verdichtet wurde. Wenn die Anzeige 13 Teil eines Mobilgeräts ist, z.B. eines Smartphones oder eines Tablets, können die Daten an ein weiteres Netzwerk 16 gesendet werden. Das Netzwerk 16 ist aber nicht zwingender Bestandteil des Assistenzsystems 12.

Bei einer Variante ist es möglich, dass eine gewisse Zeitdauer zu laufen beginnt, wenn der Bediener an einer Stelle verdichtet hat. Nach Ablauf einer vorgegebenen Zeitdauer wird dem Bediener angezeigt, dass der Beton an dieser Stelle auszuhärten beginnt und keine Verdichtung mehr möglich ist.

Bei einer anderen Weiterentwicklung kann der Bediener innerhalb dieser Zeitdauer aufgefordert werden, an dieser Stelle nochmals zu verdichten, wenn die Verdichtung an dieser Stelle nicht ausreichend war. Damit kann erreicht werden, dass der Beton noch vor seinem Aushärten verdichtet wird.

## Patentansprüche

1. System zum Führen einer Bedienperson (1) bei einer Betonverdichtung mit einer Betonverdichtungsvorrichtung (2), mit
- einer Planungsvorrichtung zum Speichern von Planungsdaten, wobei die Planungsdaten dienen zum Definieren von Orten (15) in einem definierten Gebiet (14), an denen eine Betonverdichtung mit der Betonverdichtungsvorrichtung zu erfolgen hat;
- einer Positionsbestimmungsvorrichtung zum Bestimmen der jeweils aktuellen Position der Betonverdichtungsvorrichtung (2); und mit
- einer Anzeigeeinrichtung (13) zum Anzeigen von wenigstens jeweils dem Ort (15), an dem aktuell eine Betonverdichtung zu erfolgen hat.

2. System nach Anspruch 1, wobei
- die Planungsdaten auch dienen zum Definieren einer Reihenfolge der Orte (15), in der die Betonverdichtung zu erfolgen hat; und wobei
- die Anzeigeeinrichtung (13) ausgebildet ist zum Anzeigen von wenigstens jeweils dem Ort (15) in der Reihenfolge von Orten, an dem aktuell eine Betonverdichtung zu erfolgen hat.

3. System nach einem der vorstehenden Ansprüche, wobei die Anzeigeeinrichtung (13) ausgebildet ist zum Anzeigen der Position der Betonverdichtungsvorrichtung (2) und/oder zum Anzeigen von wenigstens jeweils dem Ort (15) in der Reihenfolge von Orten, an dem als nächstes eine Betonverdichtung zu erfolgen hat.

4. System nach einem der vorstehenden Ansprüche, wobei die Anzeigeeinrichtung (13) ausgebildet ist zum Anzeigen des Ortes (15), an dem aktuell oder als nächstes eine Betonverdichtung zu erfolgen hat, in Relation zu der aktuellen Position der Betonverdichtungsvorrichtung (2).

5. System nach einem der vorstehenden Ansprüche, wobei eine Verdichtungskoordinaten-Vorgabevorrichtung vorgesehen ist, zum Erzeugen von Planungsdaten, die in der Planungsvorrichtung speicherbar sind.

6. System nach einem der vorstehenden Ansprüche, wobei
- eine Bestätigungseinrichtung vorgesehen ist, zum Bestätigen, dass an dem jeweiligen Ort, an dem aktuell eine Betonverdichtung erfolgt, eine ausreichende Betonverdichtung erreicht wurde; und wobei
- die Bestätigungseinrichtung ausgebildet ist zum Markieren des nächsten Ortes in der Reihenfolge von Orten (15), an dem als nächstes eine Betonverdichtung zu erfolgen hat.

7. System nach einem der vorstehenden Ansprüche, wobei
- die Bestätigungseinrichtung manuell von einer Bedienperson (1) betätigbar ist, um zu bestätigen, dass ausreichend verdichtet wurde; und/oder wobei
- die Bestätigungseinrichtung automatisch betätigbar ist, wenn eine Verdichtungserkennungsvorrichtung erkannt hat, dass an dem aktuellen Ort durch die Betonverdichtungsvorrichtung ausreichend verdichtet wurde.

8. System nach einem der vorstehenden Ansprüche, wobei
- die Positionsbestimmungsvorrichtung ausgebildet ist zum Bestimmen der Position einer durch eine Bedienperson (1) führbaren Rütteleinheit (3) zur Betonverdichtung; und wobei
die Positionsbestimmungsvorrichtung aufweist:
- eine Flächen-Positionsbestimmungseinrichtung mit einer Empfangseinrichtung (5), wobei die Flächen-Positionsbestimmungseinrichtung ausgebildet ist zum Bestimmen der Position der Empfangseinrichtung (5) in der Ebene;
- eine Ausrichtungsbestimmungseinrichtung (7) zum Bestimmen einer Ausrichtung (A) einer Arbeitsrichtung der Bedienperson (1); und
- eine Korrektureinrichtung zum Korrigieren der Position der Empfangseinrichtung (5) mit einem Offset (O) in Richtung der Ausrichtung (A) der Arbeitsrichtung und damit zum Bestimmen der Position (P2) der Rütteleinheit (5) in der Ebene.

9. System nach Anspruch 8, mit einer Tiefen-Positionsbestimmungseinrichtung zum Bestimmen der Position der Rütteleinheit (3) in der Tiefe.

10. System nach einem der vorstehenden Ansprüche, wobei die Tiefen-Positionsbestimmungseinrichtung eine Entfernungsmesseinrichtung aufweist, zum Messen einer Entfernung zwischen der Rütteleinheit (3) und der Empfangseinrichtung (5) der Flächen-Positionsbestimmungseinrichtung.

11. System nach einem der vorstehenden Ansprüche, mit
- einer Vergleichseinrichtung zum Vergleichen der aktuellen Position der Betonverdichtungsvorrichtung mit wenigstens dem aktuell ersten Ort der vorgegebenen Reihenfolge von Orten, an dem als nächstes eine Betonverdichtung erfolgen soll, und zum Erkennen einer Abweichung der Position der Betonverdichtungsvorrichtung von dem vorgegebenen Ort; und mit
- einer Führungseinrichtung zum Definieren einer Bewegungsmaßnahme zum Bewegen der Betonverdichtungsvorrichtung, wobei mit der Bewegungsmaßnahme die Abweichung verringerbar ist; wobei
- die Anzeigeeinrichtung ausgebildet ist zum Anzeigen der Bewegungsmaßnahme für die Bedienperson.

12. System nach einem der vorstehenden Ansprüche, mit einer Annäherungserkennungsvorrichtung zum Erkennen eines Zustands, in dem die Abweichung geringer ist als ein vorgegebener Abweichungsgrenzwert, und zum Erzeugen eines Bestätigungssignals.

13. System nach einem der vorstehenden Ansprüche, wobei die Planungsdaten außer den Daten zum Definieren der Orte, an denen eine Betonverdichtung zu erfolgen hat, weitere Daten aufweisen, ausgewählt aus der Gruppe:
- Zeitdauer der Verdichtung an dem betreffenden Ort;
- Intensität der Verdichtung an dem betreffenden Ort;
- Schwingungsanzahl der Verdichtung an dem betreffenden Ort;
- Betontyp oder Betonsorte;
- Beton-Mischungsverhältnisse,
- Daten zu Armierungseisen;
- Schütthöhe des Betons;
- Umgebungstemperatur;
- Temperatur des Betons;
- Luftfeuchte.

14. Verfahren zum Führen einer Bedienperson bei einer Betonverdichtung mit einer Betonverdichtungsvorrichtung, mit den Schritten:
- Speichern von Planungsdaten, wobei die Planungsdaten dienen zum Definieren von Orten in einem definierten Gebiet, an denen eine Betonverdichtung mit der Betonverdichtungsvorrichtung zu erfolgen hat, und zum Definieren einer Reihenfolge der Orte, in der die Betonverdichtung zu erfolgen hat;
- Bestimmen der jeweils aktuellen Position der Betonverdichtungsvorrichtung; und
- Anzeigen von wenigstens jeweils dem Ort in der Reihenfolge von Orten, an dem aktuell eine Betonverdichtung zu erfolgen hat und/oder dem Ort in der Reihenfolge von Orten, an dem als nächstes eine Betonverdichtung zu erfolgen hat.
